## Europäisches Patentamt

## European Patent Office

## Office européen des brevets

(11) Veröffentlichungsnummer: **0 161 217**

**A2**

(12) **EUROPÄISCHE PATENTANMELDUNG**

(21) Anmeldenummer: **85810207.2**

(22) Anmeldetag: **06.05.85**

(51) Int. Cl.⁴: **A 61 L 9/12**

(30) Priorität: **11.05.84 CH 2334/84**

(43) Veröffentlichungstag der Anmeldung:
**13.11.85 Patentblatt 85/46**

(84) Benannte Vertragsstaaten:
**BE CH DE FR GB IT LI LU NL SE**

(71) Anmelder: **Airwick AG**
**Postfach**
**CH-4002 Basel(CH)**

(72) Erfinder: **Mandon, Jean-Pierre**
**Bois Valrod Montamise Tronc**
**F-86360 Chasseneuil du Poitou(FR)**

(72) Erfinder: **Rapiteau, Jean**
**34 Rue des Châtaigniers**
**F-86280 Saint Benoit(FR)**

(74) Vertreter: **Schirner, Rolf et al,**
**Patentabteilung der CIBA-GEIGY AG Postfach**
**CH-4002 Basel(CH)**

(54) **Vorrichtung zur Abgabe eines flüchtigen Wirkstoffs.**

(57) Eine Vorrichtung zur Abgabe eines flüchtigen Wirkstoffs von einem Trägermaterial, welches den Wirkstoff in flüssiger Form in einem flüssigkeitsundurchlässigen Beutel enthält, wobei die Vorrichtung aus einem kreisförmigen Teil mit Stützelementen für den Träger und seitlichen Haltewangen besteht, auf denen ein weiteres kreisförmiges Teil mit zylindrischer Seitenwand, durchbrochen von seitlichen Fenstern für den Durchtritt des flüchtigen Wirkstoffs, drehbar gelagert ist. Erfindungsgemäss weist das zweite kreisförmige Teil an seiner inneren scheibenförmigen Fläche einen oder mehrere dezentral angeordnete biegsame Stifte auf, die bei erstmaligem Drehen dieses Teils auf dem ersten Teil die Haut des wirkstoffhaltigen Beutels aufreissen, so dass das Trägermaterial mit flüssigem Wirkstoff getränkt wird und somit durch die geöffneten seitlichen Fenster flüchtigen Wirkstoff abgeben kann.

Fig. 4

EP 0 161 217 A2

0161217

Airwick AG

2-14871/+/AEH

Basel (Schweiz)

Vorrichtung zur Abgabe eines flüchtigen Wirkstoffs

Gegenstand der vorliegenden Erfindung ist eine Vorrichtung zur Abgabe eines flüchtigen Wirkstoffs, beispielsweise für die Luftverbesserung oder für baktrizide oder insektizide Zwecke.

Viele der bisher bekannten Abgabevorrichtungen für flüchtige Wirkstoffe gehen von mit flüssigem Wirkstoff getränkten Trägern aus einem saugfähigen oder porösen Material aus. Das hat den Nachteil, dass bis zum erstmaligen Gebrauch der Vorrichtung je nach Dauer der vorherigen Lagerung beim Hersteller oder Händler ein beträchtlicher Teil des Wirkstoffs sich bereits verflüchtigt hat.

Die FR-PS 1 231 135 beschreibt eine Vorrichtung zur Abgabe flüchtiger Substanzen, bestehend aus einem porösen Träger, der einen flüssigen Wirkstoff in einem flüssigkeitsundurchlässigen Beutel enthält und mit einer Metallfolie abgedeckt ist. Durch Pressen auf die Metallfolie wird der Beutel zerquetscht oder mittels zwischen Beutel und Träger gelagerter spitzer oder kantiger Metallstücke perforiert, so dass der flüssige Wirkstoff ausfliesst und vom porösen Träger aufgesogen wird, um allmählich verflüchtigt an die Umgebungsluft abgegeben zu werden. Nachteilig bei dieser Vorrichtung ist die fehlende Regulierbarkeit der Abgabe des flüchtigen Wirkstoffs.

Die Aufgabe der vorliegenden Erfindung war es daher, eine Vorrichtung zu schaffen, welche in der Lage ist, sowohl die vorzeitige Verflüchtigung des Wirkstoffs zu verhindern als auch die Abgabe von flüchtigem Wirkstoff an die Umgebungsluft je nach Bedarf zu regulieren.

Diese Aufgabe wird gelöst durch die erfindungsgemässe Vorrichtung zur Abgabe eines flüchtigen Wirkstoffs von einem Trägermaterial, welches den Wirkstoff in flüssiger Form in einem flüssigkeitsundurchlässigen Behälter enthält, bestehend aus einem kreisförmigen Teil 4 mit Stützelementen für den Träger und seitlichen Haltewangen, auf denen ein anderes kreisförmiges Teil 1 mit zylindrischer Seitenwand 2, durchbrochen von seitlichen Fenstern 3 für den Durchtritt des flüchtigen Wirkstoffs, drehbar gelagert ist, dadurch gekennzeichnet, dass das Teil 1 an seiner inneren scheibenförmigen Fläche einen oder mehrere dezentral angeordnete biegsame Stifte 9 aufweist, die in der Lage sind, die Haut des wirkstoffhaltigen Behälters durch eine Drehbewegung der Teile 1 und 4 gegeneinander aufzureissen, um so das Trägermaterial durch Tränken mit flüssigem Wirkstoff zu aktivieren.

Vorzugsweise sind die biegsamen Stifte schräg zur Drehrichtung vom Teil 1 zum Teil 4 an der inneren scheibenförmigen Fläche des Teils 1 angeordnet. Die biegsamen Stifte können an ihrem äusseren Ende Schneidkanten oder Widerhaken aufweisen, die bei der ersten Drehbewegung der beiden Teile 1 und 4 gegeneinander mit ihrer Spitze im Trägermaterial hängen bleiben und sich in die Haut des wirkstoffhaltigen Behälters bohren, um sie aufzureissen. Zum besseren Hängenbleiben im Trägermaterial können die Schneidkanten an der äussersten Stelle mit einem Dorn versehen sein.

Eine Vorrichtung der vorgenannten Art wird durch die Figuren 1 bis 9 wie folgt dargestellt:

Figur 1 ist eine Perspektivzeichnung mit geschlossenen Seitenfenstern,

Figur 2 zeigt die Vorrichtung von der Seite gesehen, ebenfalls in
       geschlossenem Zustand, und
Figur 3 zeigt sie von der Seite gesehen in geöffnetem Zustand.


Die Figuren 4 bis 9 bringen eine Wiedergabe der Vorrichtung gegenüber derjenigen der Figuren 1 bis 3 in etwa doppelter Vergrösserung,
wobei


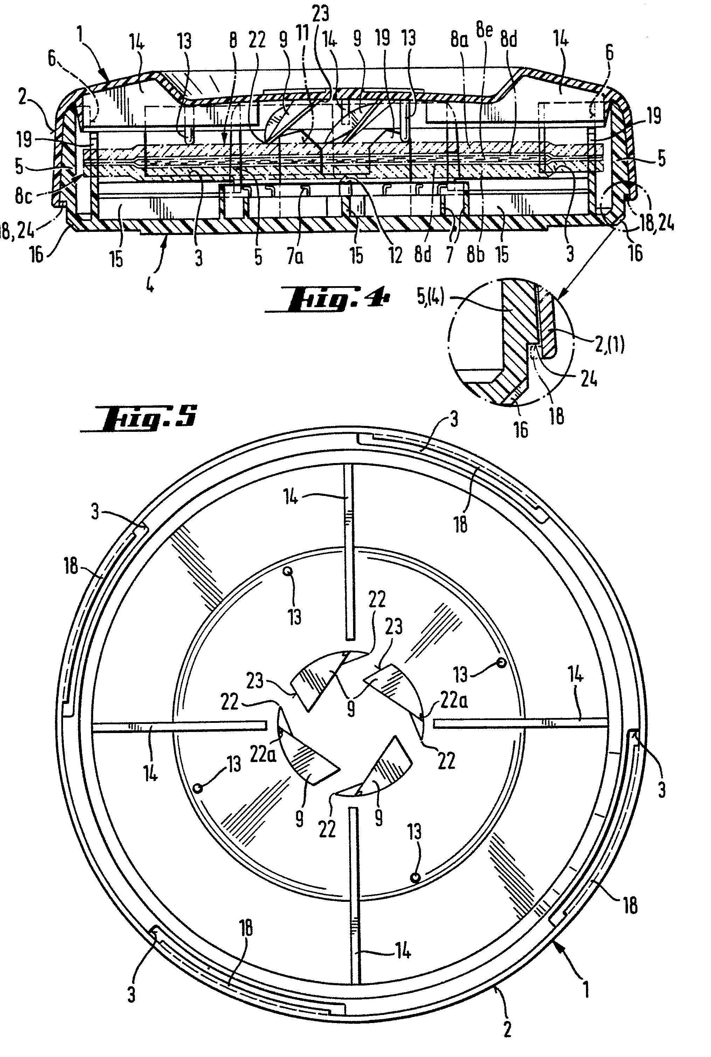

Figur 4 einen Vertikalschnitt durch die gesamte Vorrichtung mit noch
       nicht aktiviertem Trägermaterial,
Figur 5 eine Aufsicht auf die innere scheibenförmige Fläche des
       Teils mit den seitlichen Fenstern und vier erfindungsgemässen dezentral angeordneten, pflugscharartig ausgebildeten
       biegsamen Stiften,

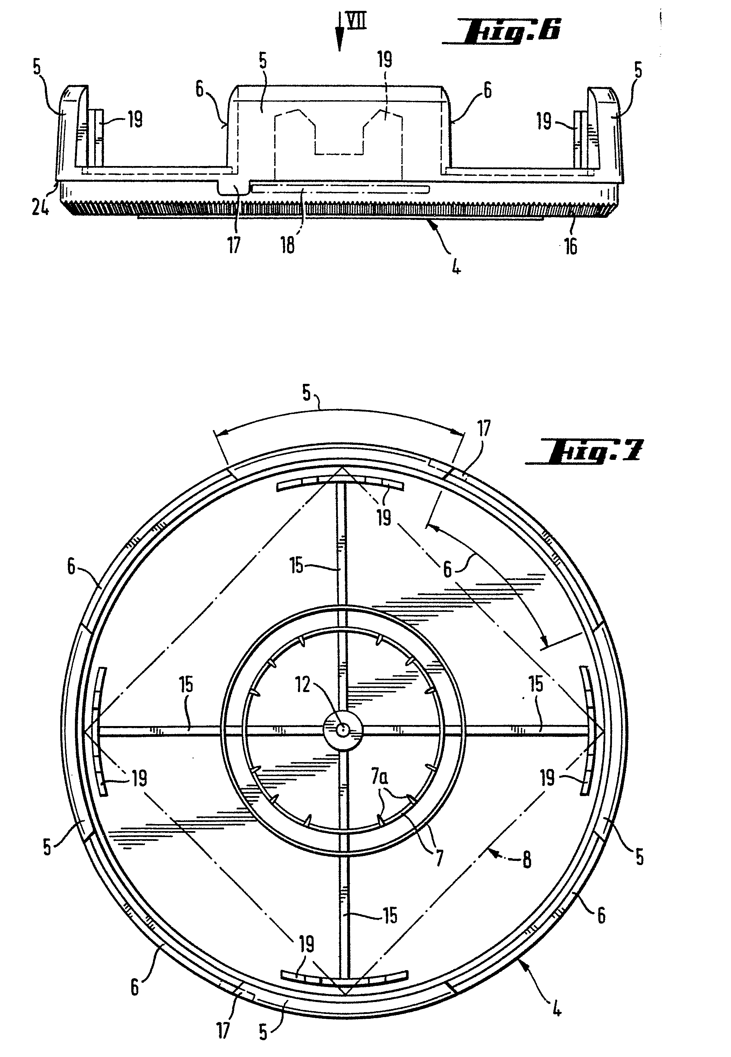

Figur 6 eine Seitenansicht des kreisförmigen Teils mit den seitlichen Haltewangen,
Figur 7 eine Aufsicht auf dessen innere scheibenförmige Fläche in
       Richtung VII zur Figur 6 mit strichpunktiert angedeutetem
       Trägermaterial,

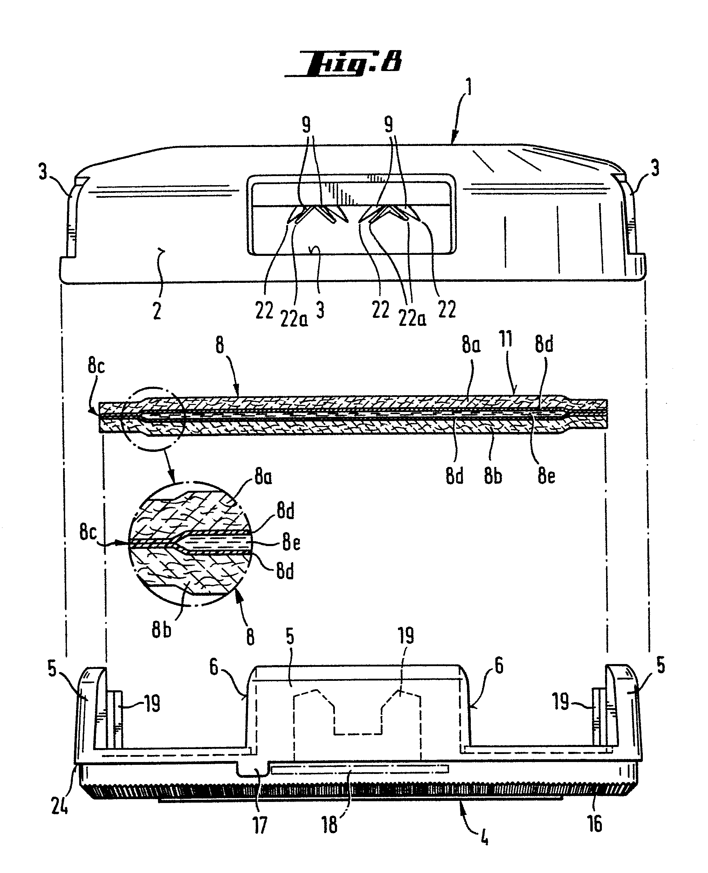

Figur 8 eine Seitenansicht aller Teile als Explosionszeichnung und

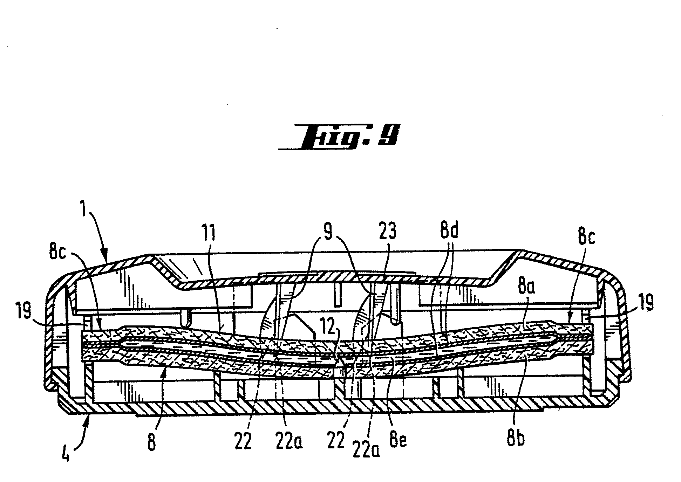

Figur 9 einen Vertikalschnitt entsprechend Figur 4, jedoch mit
       aktiviertem Trägermaterial
darstellen.


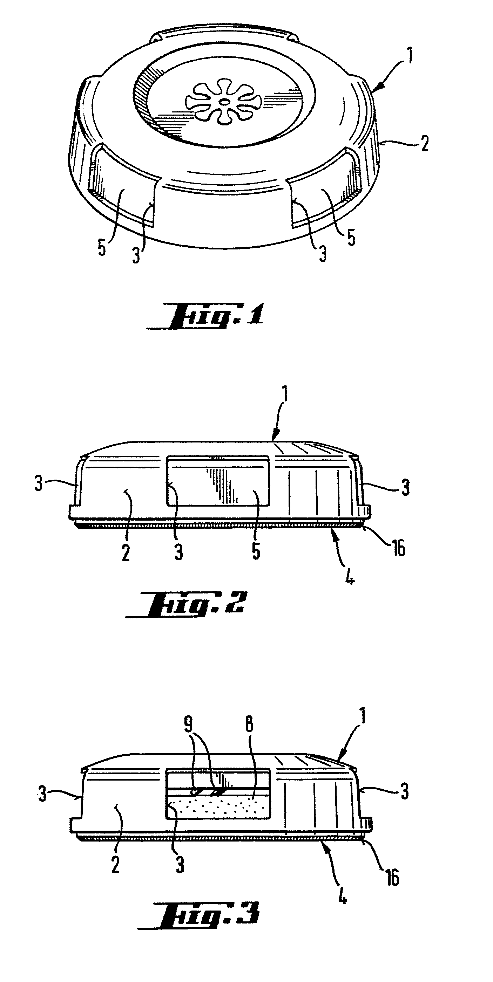

Im einzelnen zeigen die Figuren 1 bis 9, dass die Vorrichtung zur
Abgabe eines flüchtigen Wirkstoffs aus zwei ineinander dreh- und
verschliessbaren kreisförmigen Teilen 1 und 4 und dem Trägermaterial 8  besteht. Das Teil 1 hat an seiner ringförmigen Seitenwand 2 mehrere Fenster 3, welche durch die Haltewangen 5 des Teils 4
teilweise oder ganz verdeckt werden können. Durch die Verdrehbarkeit
der Teile 1 und 4 ineinander lässt sich eine stufenlose Verstellung
der Fenstergrösse von 3 bewirken. Zwischen den einzelnen Haltewangen 5  des Teil 4 befinden sich Oeffnungen 6, wobei die Bereiche
von 5 und 6 jeweils etwa gleich lang sind, wie Figur 7 zeigt.
Dadurch wird sichergestellt, dass eine möglichst grosse Gehäuseöffnung und damit -belüftung erreicht wird.

Auf der inneren scheibenförmigen Fläche des Teils 4 befinden sich,
wie besonders Figuren 4 und 7 zeigen, Stützelemente 7 und 12 für das
Trägermaterial 8, welches den Wirkstoff in einem flüssigkeitsundurchlässigen Behälter enthält, den es in kompakter Form umschliesst. Das auf den Stützelementen 7 und 12 liegende Trägermaterial 8 ist, wenn es in quadratischer Form vorliegt, gegen seitliche Verschiebungen mit seinen Ecken in den Ausnehmungen der
Rippen 19 fixiert, die sich parallel zu den Haltewangen 5 auf der
inneren scheibenförmigen Fläche des Teils 4 befinden. Für die
Niederhaltung des Trägermaterials 8 auf den Stützelementen 7 und 12
sorgen die senkrechten Stifte 13 im Teil 1, wie Figur 4 zeigt.

In den Figuren 4, 5 und 9 sind die erfindungsgemässen dezentral
angeordneten biegsamen Stifte 9 an der inneren scheibenförmigen
Fläche des Teils 1 erkennbar. Sie sind scharnierartig bzw. flexibel
bei 23 mit der Innenseite von Teil 1 verbunden und vorzugsweise mit
diesem in einem Guss aus thermoplastischem Material hergestellt. In
der dargestellten schrägen Form der Stifte 9 sind diese am äusseren
Ende mit Schneidkanten 22 versehen, die jeweils an der äussersten
Stelle einen Dorn 22a aufweisen können, der bei der Drehbewegung von
Teil 1 im Teil 4 entgegen dem Uhrzeigersinn in der Aussenschicht 11
des Trägermaterials 8, 8a hängen bleibt. Durch das Hängenbleiben
biegen sich die Stifte 9 gegen die Drehbewegung, wodurch die am Ende
der Stifte 9 befindlichen Schneidkanten 22 durch das Trägermaterial
8, 8a und in die Haut des flüssigkeitsundurchlässigen Behälters 8d
dringen und sie verletzen. Der flüssige Wirkstoff 8e fliesst nun aus
dem Behälter 8d und tränkt das Trägermaterial 8, 8a, 8b, von dem
er durch die seitlichen Fenster 3 in flüchtiger Form an die Umgebungsluft abgegeben werden kann. Als Trägermaterial 8 kommen Papier,
Pappkarton, vlies- oder schwammartiges Material aus Kunststoff oder
dergleichen in Betracht, welches sich mit flüssigen Wirkstoffen oder
wirkstoffhaltigen Lösungsmitteln imprägnieren lässt. Vorzugsweise
ist das Trägermaterial aus zwei Lagen 8a, 8b gebildet, welches in sich, über Randschweissungen 8c, einen zweilagigen flüssigkeitsundurchlässigen Behälter 8d aus Folienmaterial mit einer Wirkstofffüllung 8e einschliesst (Fig. 8).

Die Teile 1 und 4 werden im allgemeinen als einstückiger Formling
aus Kunststoffmaterial hergestellt. Zwar werden diese Teile vorzugsweise aus Polyäthylen, Polypropylen oder Polyvinylchlorid hergestellt, jedoch versteht es sich, dass man auch andere Kunststofftypen verwenden kann und dass die beiden Teile dabei aus demselben
oder aus verschiedenartigen Kunststoffmaterialien geformt werden
können.

Die in den Figuren 1 bis 9 gezeigte Vorrichtung stellt nur eine von
vielen möglichen Ausführungsarten dar. Die äussere Form derselben
ist bereits Gegenstand verschiedener Schutzrechte, beispielsweise
vom spanischen Gebrauchsmuster Nr. 261,291. Im wesentlichen soll
gezeigt werden, wie die erfindungsgemässen dezentral angeordneten
biegsamen Stifte zum Verletzen bzw. Aufreissen der Haut des wirkstoffhaltigen Behälters im Trägermaterial und damit dessen Aktivierung durch Imprägnieren mit Wirkstoff erst unmittelbar vor der
ersten Benutzung der Vorrichtung aussehen kann. Die Anzahl der
Stifte ist nicht beschränkt, es kann sich beispielsweise auch nur um
einen handeln. Auch die Form der Stifte ist variabel, wobei deren
einzige Aufgabe darin besteht, beim erstmaligen Oeffnen der seitlichen Fenster das Trägermaterial zu aktivieren, um vorzeitige
Verluste durch Verflüchtigung des Wirkstoffs zu verhindern.

Weitere Teile in den Figuren können zum Beispiel Versteifungsrippen
14 und 15 an den Teilen 1 und 4 sein, eine Riffelung 16 am Teil 4,
welche die Handhabung beim Oeffnen und Schliessen der Vorrichtung
erleichtert, Verdrehanschläge 17 am Teil 4 und Uebergriffsleisten 18 am Teil 1, welche den Weg des Verdrehens der Teile 1 und
4 zueinander begrenzen, wozu auch das Schulterteil 24 dient.
Schliesslich kann auch der zentral im Teil 4 angeordnete Dorn 12 die
Aufgabe als Fixierungselement statt als Stützelement haben, indem er
mit dem verlängerten oberen Teil durch eine im Trägermaterial befindliche flüssigkeitsdichte zentrale Oeffnung ragt, wobei der wirkstoffhaltige Behälter die Form eines Rings oder dergleichen um die Oeffnung herum hat. Eine zusätzliche Fixierung des Trägermaterials kann durch kurze Stifte 7a im Stützring 7 erfolgen, wie Figur 7 zeigt.

Bei Nichtvorliegen einer zentralen Oeffnung im Trägermaterial kann der Dorn 12 auch als Element zur Verletzung der Folie des wirkstoffhaltigen Behälters dienen, indem beispielsweise bei der Aktivierung durch die biegsamen Stifte 9 das Trägermaterial nach unten gebogen und dabei auf den Dorn 12 gedrückt wird, wodurch dieser in den Behälter 8d eindringt (Figur 9).

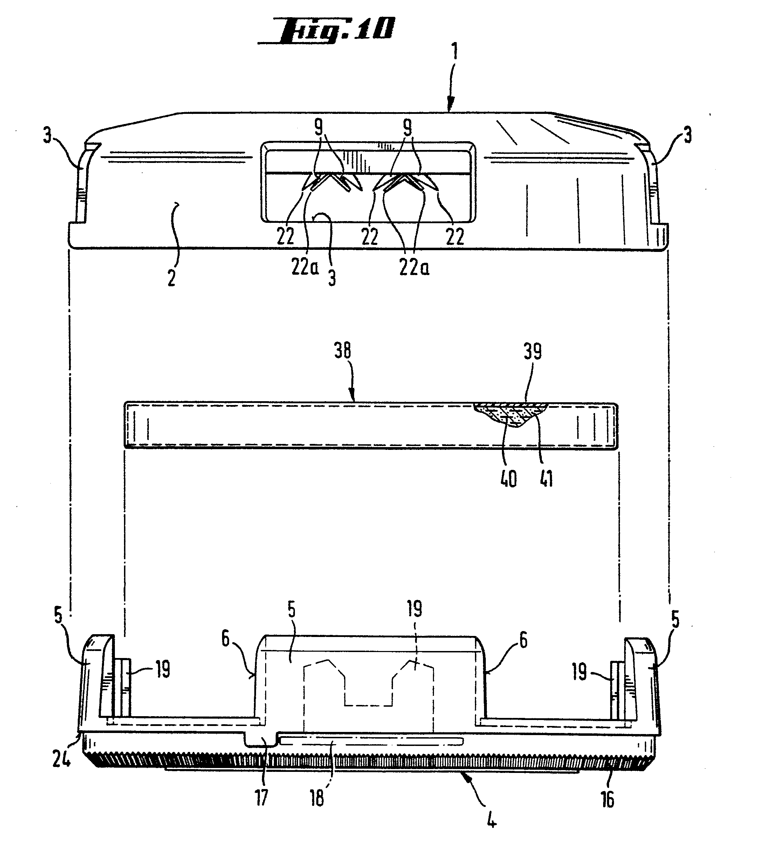

Eine andere Form von Wirkstoff enthaltenen Behältern 38, welcher mit der Vorrichtung nach Figuren 1 bis 9 verwendet werden kann, zeigt Figur 10. Hier ist das Trägermaterial 41 bereits mit Wirkstoff 40 getränkt. Die Schneiden 22 der Stifte 9 zerstören seine äussere wirkstoffundurchlässige Umhüllung 39 derart, dass darin Oeffnungen entstehen, durch die der Wirkstoff verdunsten kann.

Die Vorrichtung ist für die regulierbare Abgabe verflüchtigter Mengen von in einem Trägermaterial enthaltenem flüssigen Wirkstoff vorgesehen. Vorzugsweise steht dabei die räumliche Verteilung eines Luftbehandlungsstoffs im Vordergrund. Es lassen sich jedoch in gleicher Weise auch andere flüchtige Wirkstoffe verteilen, wie z.B. Insektizid- oder Bakterizidstoffe.

0161217

Patentansprüche

1. Vorrichtung zur Abgabe eines flüchtigen Wirkstoffs von einem Trägermaterial, welches den Wirkstoff in flüssiger Form in einem flüssigkeitsundurchlässigen Behälter enthält, bestehend aus einem kreisförmigen Teil (4) mit Stützelementen für den Träger und seitlichen Haltewangen, auf denen ein anderes kreisförmiges Teil (1) mit zylindrischer Seitenwand (2), durchbrochen von seitlichen Fenstern (3) für den Durchtritt des flüchtigen Wirkstoffs, drehbar gelagert ist, dadurch gekennzeichnet, dass das Teil (1) an seiner inneren scheibenförmigen Fläche einen oder mehrere dezentral angeordnete biegsame Stifte (9) aufweist, die in der Lage sind, die Haut des wirkstoffhaltigen Behälters durch eine Drehbewegung der Teile (1) und (4) gegeneinander aufzureissen, um so das Trägermaterial durch Tränken mit flüssigem Wirkstoff zu aktivieren.

2. Vorrichtung gemäss Patentanspruch 1, dadurch gekennzeichnet, dass die biegsamen Stifte (9) schräg zur Drehrichtung vom Teil (1) zum Teil (4) an der inneren scheibenförmigen Fläche des Teils (1) angeordnet sind.

3. Vorrichtung gemäss Patentanspruch 1 oder 2, dadurch gekennzeichnet, dass die biegsamen Stifte (9) an ihrem äusseren Ende Schneidkanten (22) oder Widerhaken aufweisen.

4. Vorrichtung gemäss Patentanspruch 3, dadurch gekennzeichnet, dass die Schneidkanten (22) an der äussersten Stelle mit einem Dorn versehen sind.

5. Vorrichtung gemäss Patentanspruch 1, dadurch gekennzeichnet, dass das kreisförmige Teil (4) im Zentrum einen Dorn (12) als Stütz-, Fixierungs- oder Durchbohrungselement aufweist.

FO 7.1/SI/eg*

0161217

_Fig. 1_

_Fig. 2_

_Fig. 3_

**Fig. 4**

**Fig. 5**

**Fig. 6**

**Fig. 7**

## Fig.8

Fig. 9

**Fig. 10**